# EUROPÄISCHE PATENTANMELDUNG

(11) **EP 1 632 261 A1**
(43) Veröffentlichungstag der Anmeldung: **08.03.2006**
(21) Anmeldenummer: 05016839.2
(22) Anmeldetag: 03.08.2005
(51) Int. Cl.: A61M 16/00, A61M 16/20, A62B 7/02, A62B 9/02

(54) **Sauerstoffversorgungseinrichtung**

(30) Priorität: 02.09.2004 DE 102004042388
(71) Anmelder: Dräger Aerospace GmbH, 23560 Lübeck (DE)
(72) Erfinder: Mantey, Carsten, 23820 Wulfsfelde (DE)
(74) Vertreter: Hemmer, Arnd

(57) **Zusammenfassung**

Sauerstoffversorgungsvorrichtung für eine Atemmaske (2) mit mindestens einem Sauerstoffdruckspeicher (20, 20'), der über ein steuerbares Absperrventil (34) mit einem Zwischenspeicher (36) leitungsverbunden ist, an den ein die Maske (2) versorgender Impulsatemregler (8) anschließt, wobei eine elektronische Steuerung (18) vorgesehen ist, welche das Absperrventil (34) in Abhängigkeit des im Zwischenspeicher (36) herrschenden Drucks steuert.

## Beschreibung

Die Erfindung betrifft eine Sauerstoffversorgungsvorrichtung für eine Atemmaske mit den im Oberbegriff des Anspruchs 1 angegebenen Merkmalen.

Aus der Medizintechnik sind Sauerstoffversorgungssysteme bekannt, die dem Benutzer einer Atemmaske zu Beginn des Einatmens eine dosierte Menge Sauerstoff, das so genannte Bolusvolumen und daran anschließend die Umgebungsluft als Atemgas zur Verfügung stellen. Hierzu weisen diese Sauerstoffversorgungssysteme eine Impulsatemreglereinheit auf. In dieser betätigt ein Drucksensor beim Einatmen ein in der Versorgungsleitung zwischen einer Sauerstoffquelle und der Atemmaske angeordnetes Magnetventil, welches das Bolusvolumen an Sauerstoff freigibt. Als Sauerstoffquelle werden Druckgasflaschen eingesetzt, in denen der Sauerstoff gespeichert ist. Um den Druck in den Druckgasflaschen, der bis zum 200 Bar betragen kann, abzusenken, ist dem Absperrventil jeweils einer Druckgasflasche ein gängiger Druckminderer nachgeschaltet, wie er üblicherweise zur Druckabsenkung an Druckgasflaschen Verwendung findet.

Ein Nachteil dieser mechanisch ausgebildeten Druckminderer ist deren relativ große Störanfälligkeit. Insbesondere die in den Druckminderen verwendeten Diaphragmen und Federn können bei den im Druckminderer ablaufenden Regelvorgängen und den damit verbundenen Druckwechseln leicht beschädigt oder zerstört werden, was zu einem Totalausfall des Druckminderers und damit zu einem Totalausfall des Sauerstoffversorgungssystems führen kann. Daneben sind Fälle bekannt, in denen die in dem Druckminderer ablaufenden Druckwechsel in diesen sich aufschaukelnde Bauteilschwingungen hervorgerufen haben, die zum Versagen des Druckminderers geführt haben. Ferner besteht die Gefahr, dass eindringender Staub und andere Verunreinigungen die Funktionsfähigkeit dieser Druckminderer beeinträchtigen bzw. zum Ausfall dieser Geräte führen. Werden die oben beschriebenen Sauerstoffversorgungssysteme beispielsweise in der Luftfahrt, im Bergbau, von Fallschirmspringern oder Bergsteigern benutzt, erweisen sich das relativ große Gewicht und die ebenfalls verhältnismäßig großen Abmessungen mechanischer Druckminderer als nachteilig.

Der vorliegenden Erfindung liegt nun die Aufgabe zugrunde, eine Sauerstoffversorgungsvorrichtung zu schaffen, die eine hohe Funktionssicherheit aufweist und dabei möglichst raum- und gewichtssparend ausgebildet ist. Ferner soll die Sauerstoffversorgungsvorrichtung einfach und kostengünstig herzustellen sein.

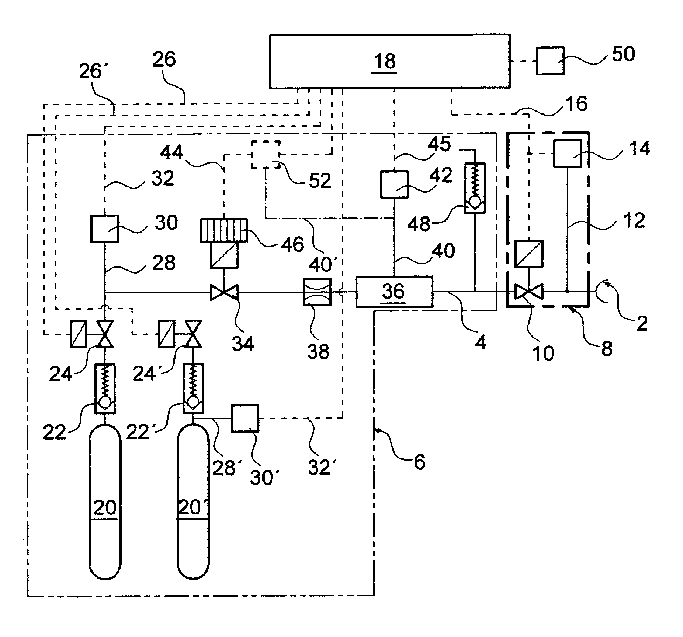

Diese Aufgabe wird durch eine Sauerstoffversorgungsvorrichtung mit den im Anspruch 1 angegebenen Merkmalen gelöst. Vorteilhafte Weiterbildungen der Erfindung ergeben sich aus den Unteransprüchen, der nachfolgenden Beschreibung und der Zeichnung.

Die erfindungsgemäße Sauerstoffversorgungsvorrichtung für eine Atemmaske weist mindestens einen Sauerstoffdruckspeicher auf. Dieser ist über ein steuerbares Absperrventil mit einem Zwischenspeicher leitungsverbunden, an den ein die Atemmaske versorgender Impulsatemregler anschließt. Dabei ist eine elektronische Steuerung vorgesehen, welche das Absperrventil in Abhängigkeit des im Zwischenspeicher herrschenden Drucks steuert.

Ähnlich bekannten Sauerstoffversorgungssystemen weist die erfindungsgemäße Sauerstoffversorgungsvorrichtung eine mit einem Sauerstoffdruckspeicher leitungsverbundene Atemmaske auf, wobei der Atemmaske ein Impulsatemregler zum Freigeben eines Bolusvolumens an Sauerstoff vorgeschaltet ist.

Bei der erfindungsgemäßen Sauerstoffversorgungsvorrichtung sind in der Leitungsverbindung zwischen Sauerstoffdruckspeicher und Impulsatemregler ein steuerbares Absperrventil mit einem sich daran anschließenden Zwischenspeicher sowie eine elektronische Steuerung zum Steuern des Absperrventils vorgesehen. Die elektronische Steuerung veranlasst zunächst das Öffnen des Absperrventils. Dadurch wird der Zwischenspeicher mit Sauerstoff gefüllt. Erreicht der Druck in dem Zwischenspeicher einen Sollwert, der den Atmungsbedingungen des Nutzers der Atemmaske angepasst ist, wird das Absperrventil von der elektronischen Steuerung geschlossen. Wird der Impulsatemregler nun durch einen Impuls beim Einatmen aktiviert, stellt der Impulsatemregler dem Benutzer der Atemmaske den in dem Zwischenspeicher gespeicherten Sauerstoff als Bolusvolumen zur Verfügung. Der Zwischenspeicher entleert sich ganz oder teilweise, wobei die Steuerung das Absperrventil wieder öffnet und sich der eben beschriebene Vorgang wiederholen kann. Das Öffnen und Schließen des Absperrventils ist grundsätzlich unabhängig von der Entnahmezeit, kann also auch in sehr kurzen Steuerzeiten liegen.

Auf diese Weise kann bei der erfindungsgemäßen Sauerstoffversorgungsvorrichtung auf den sonst üblichen mechanischen Druckminderer verzichtet werden, da durch gezieltes Ansteuern des Absperrventils durch die elektronische Steuerung eine gegenüber dem Druck im Sauerstoffdruckspeicher deutliche Druckminderung im Zwischenspeicher auf einen Druck von beispielsweise 4 bar realisierbar ist. Das Speichervolumen des Zwischenspeichers ist frei wählbar und kann beispielsweise das größte zu erwartende Bolusvolumen an Sauerstoff beinhalten.

Die erfindungsgemäße Sauerstoffversorgungsvorrichtung kann erheblich leichter und kompakter als bekannte Vorrichtungen dieser Art ausgebildet sein, was sich besonders dann als Vorteil erweist, wenn diese Sauerstoffversorgungsvorrichtung beispielsweise in Segel- und Militärflugzeugen mit begrenztem Raumangebot zum Einsatz kommt oder wenn sie mobil als tragbare Sauerstoffversorgungsvorrichtung eingesetzt wird, beispielsweise im Bergbau, beim Fallschirmspringen oder Bergsteigen. Ferner ist durch die Minimierung der mechanischen Bauteile die Störanfälligkeit der erfindungsgemäßen Sauerstoffversorgungsvorrichtung gegenüber bekannten anderen Sauerstoffversorgungssystemen erheblich verringert.

Zweckmäßigerweise ist die elektronische Steuerung des steuerbaren Absperrventils zwischen Sauerstoffdruckspeicher und Zwischenspeicher Teil einer elektronischen Steuer- und Regelvorrichtung, welche auch ein Absperrventil des Impulsatemreglers steuert.

Zwischen dem Zwischenspeicher und der Atemmaske ist ein Absperrventil, vorzugsweise ein Magnetventil, des Impulsatemreglers angeordnet, das kurzzeitig öffnet, um das in dem Zwischenspeicher gespeicherte Bolusvolumen zur Atemmaske freizugeben. Zum Betätigen dieses Absperrventils und zu dessen Energieversorgung ist eine elektronische Steuerung vorzusehen. Diese elektronische Steuerung kann vorteilhaft mit der elektronischen Steuerung des Absperrventils zum Befüllen des Zwischenspeichers in einer elektronischen Steuer- und Regelvorrichtung zusammengefasst werden. Auch diese Maßnahme verringert die Baugröße der Sauerstoffversorgungsvorrichtung.

In einer Weiterbildung der erfindungsgemäßen Sauerstoffversorgungsvorrichtung ist es vorgesehen, dass die elektronische Steuer- und Regelvorrichtung außerdem den Füllstand eines angeschlossenen Sauerstoffdruckspeichers überwacht und diesen Füllstand beispielsweise auf einem Display darstellt oder bei einem leeren Sauerstoffdruckspeicher automatisch auf einen weiteren noch gefüllten Sauerstoffdruckspeicher umschaltet, der auch in der Sauerstoffversorgungsvorrichtung eingebunden ist.

Es ist ebenfalls denkbar, dass der Befüllungsgrad des eingesetzten Sauerstoffspeichers auf einem Display der Steuer- und Regeleinrichtung dargestellt wird und der Benutzer der Sauerstoffversorgungsvorrichtung aufgrund der Displayanzeige oder eines von der Steuer- und Regeleinrichtung aktivierten akustischen Signals manuell von einem leeren auf einen gefüllten Sauerstoffspeicher umschaltet.

Bevorzugt stellt die Steuer- und Regeleinrichtung den Soll-Druck des Zwischenspeichers höhen- oder druckabhängig ein.

Mit zunehmender Höhe sinkt der Luftdruck und dementsprechend der Partialdruck des in der Luft enthaltenden Sauerstoffs, so dass beim Einatmen von Umgebungsluft in sehr großer Höhe ein deutlicher Sauerstoffmangel auftritt. Insbesondere bei Sauerstoffversorgungsanlagen, die im Bereich der Luftfahrt Verwendung finden, ist es daher nötig, das durch die Sauerstoffversorgungsvorrichtung zugeführte Bolusvolumen an Sauerstoff erhöhen zu können, um den höhenbedingten Sauerstoffmangel ausgleichen zu können. Daher ist es vorteilhaft, die aus dem Zwischenspeicher austretende Sauerstoffmenge über die Erhöhung des Speicherdrucks zu vergrößern, um dem Impulsatemregler den zusätzlich benötigten Sauerstoff bei gleicher Ventilöffnungszeit zur Verfügung stellen zu können. Die elektronische Steuer- und Regeleinrichtung stellt den Soll-Druck des Zwischenspeichers entsprechend unter Berücksichtigung der Höhe oder des Umgebungsdrucks ein.

Zusätzlich bzw. alternativ stellt die Steuer- und Regeleinrichtung vorteilhaft die Schaltzeit des Absperrventils des Impulsatemreglers höhen- oder umgebungsdruckabhängig ein. So wird die Öffnungszeit des Absperrventils zweckdienlich so verlängert, dass eine höhenabhängig größere Sauerstoffmenge, die in dem Zwischenspeicher gespeichert ist, der Atemmaske als Bolusvolumen zugeführt werden kann.

Für das Absperrventil ist vorteilhaft eine vorzugsweise manuell betätigbare Notsteuereinrichtung vorgesehen, die das Absperrventil taktweise öffnet und schließt.

Die Notsteuereinrichtung für das Absperrventil ist für den Fall vorgesehen, dass die elektronische Steuerung des Absperrventils nicht einwandfrei arbeitet bzw. ausfällt, so dass der Sauerstoff dem Zwischenspeicher nicht in ausreichendem Maße, gar nicht oder mit zu hohem Druck zugeführt werden kann. Bei der Notsteuereinrichtung handelt es sich vorzugsweise um einen mechanischen Triggermechanismus, in dem, ähnlich wie bei einem mechanischen Kurzzeitmesser, eine aufgezogene Spiralfedermechanik vorgesehen ist, die dann ein taktweises Öffnen und Schließen des Absperrventils bewirkt.

Vorteilhaft ist der Zwischenspeicher mit einem Drucksensor leitungsverbunden. Dieser Drucksensor ermittelt den Sauerstoffdruck in dem Zwischenspeicher und stellt in Verbindung mit der elektronischen Steuer- und Regeleinrichtung das Absperrventil bei Erreichen eines voreingestellten Soll-Drucks so, dass es die Leitungsverbindung zu dem Zwischenspeicher sperrt und nach einem Druckabbau in dem Zwischenspeicher wieder öffnet.

Es kann auch zweckmäßig sein, statt des Drucksensors einen Druckschalter vorzusehen, der das Absperrventil bei Erreichen des Soll-Drucks sperrend stellt und nach einer Sauerstoffentnahme aus dem Zwischenspeicher wieder öffnet. Der Druckschalter ist mit der Steuer- und Regeleinrichtung leitungsverbunden, so dass von der Steuer- und Regeleinrichtung über eine Steuerleitung der den Druckschalter auslösende Sauerstoff-Soll-Druck des Zwischenspeichers an dem Druckschalter einstellbar bzw. veränderbar ist und so das Speichervolumen des Zwischenspeichers beispielsweise einer Höhenänderung angepasst werden kann.

Besonders günstig ist es, wenn an dem Sauerstoffdruckspeicher ebenfalls ein Drucksensor angeordnet ist. Dieser Drucksensor ermittelt den Druck und damit den Befüllungsgrad des Sauerstoffdruckspeichers. Steht der Drucksensor in Verbindung mit der elektronischen Steuer- und Regeleinrichtung, kann diese in Abhängigkeit der von dem Drucksensor übermittelten Werte gegebenenfalls einen leeren Sauerstoffspeicher erkennen und auf einen weiteren, ebenfalls in der Sauerstoffversorgungsvorrichtung vorgesehenen, befüllten Sauerstoffdruckspeicher umschalten.

Vorteilhaft ist zwischen dem Zwischenspeicher und dem Impulsatemregler ein Überdruckventil angeordnet. Dieses Überdruckventil ist dafür vorgesehen, wenn der Sauerstoffdruck in dem Zwischenspeicher einen maximal zulässigen Wert beispielsweise durch eine fehlerhaft arbeitende elektronische Steuerung des Absperrventils überschreitet. Der Überdruck kann dann durch das Überdruckventil abgeführt werden, so dass der Impulsatemregler nicht durch einen unzulässig hohen Druck beschädigt wird und/oder der Sauerstoffdruck in der Atemmaske den hier zulässigen Wert überschreitet.

Zweckmäßigerweise ist in der Leitungsverbindung zwischen dem Absperrventil und dem Zwischenspeicher eine Drosselstelle angeordnet. Diese Drosselstelle ist vorzugsweise einstellbar ausgebildet. Sie dient z. B. dazu, den Volumenstrom an Sauerstoff, der bei Ausfall der elektronischen Steuerung des Absperrventils von der Notsteuereinrichtung taktweise von dem Absperrventil freigegeben wird, so einzustellen, dass bei einem gleichzeitig geöffneten Absperrventil des Impulsatemreglers ein konstanter Sauerstoffstrom an der Atemmaske anliegt. Des Weiteren kann die Drossel durch Einstellen des Drosselquerschnitts und damit der Füllzeit des Zwischenspeichers an unterschiedliche Atemfrequenzen angepasst werden, so dass beispielsweise auch einem hastig atmenden Benutzer der Atemmaske bei jedem Atemzyklus das Bolusvolumen an Sauerstoff zur Verfügung gestellt wird.

Bevorzugt ist an der elektronischen Steuer- und Regeleinrichtung ein Höhenmesser angebunden. Hierbei handelt es sich vorzugsweise um ein Barometerelement, welches insbesondere bei Nutzung der Sauerstoffversorgungsvorrichtung in großer Höhe den Wert des Umgebungsdrucks an die elektronische Steuer- und Regeleinrichtung weiterleitet. Auf der Grundlage dieser Werte stellt die Steuer- und Regeleinrichtung dann, wie oben beschrieben, den Soll-Druck des Zwischenspeichers und/oder die Schaltzeit des Absperrventils des Impulsatemreglers höhengerecht ein.

Nachfolgend wird die Erfindung anhand eines in der Figur dargestellten Ausführungsbeispiels näher erläutert, wobei die Figur eine Schaltskizze einer erfindungsgemäßen Sauerstoffversorgungsvorrichtung zeigt.

Eine Atemmaske 2 ist über eine Versorgungsleitung 4 an einer Sauerstoffversorgungseinheit 6 angebunden. Dabei ist der Atemmaske 2 eine Impulsatemregelung 8 vorgeschaltet.

Die Impulsatemregelung 8 weist ein in der Versorgungsleitung 4 angeordnetes elektrisch steuerbares Magnetventil 10 auf. In einer unbeschalteten Stellung verschließt dieses die Versorgungsleitung 4. Zwischen dem Magnetventil 10 und der Atemmaske 2 zweigt von der Versorgungsleitung 4 eine Leitung 12 ab, an der ein Drucksensor 14 angeschlossen ist. Über eine elektrische Steuerleitung 16 steht das Magnetventil 10 mit einer elektronischen Steuer- und Regeleinheit 18 in Verbindung. Der Drucksensor 14 ist ebenfalls mit der elektronischen Steuer- und Regeleinheit 18 signalverbunden.

Durch das Einatmen erzeugt ein Benutzer der Atemmaske 2 in der Versorgungsleitung 4 zwischen der Atemmaske 2 und dem geschlossenen Magnetventil 10 einen Unterdruck, der von dem Drucksensor 14 detektiert wird. Die elektronische Steuer- und Regeleinheit 18 veranlasst, nachdem sie von dem Drucksensor 14 ein entsprechendes Unterdrucksignal erhalten hat, ein zeitlich begrenztes Öffnen des Magnetventils 10, sodass Sauerstoff von der Sauerstoffversorgungseinheit 6 zu der Atemmaske 2 strömen kann. Die Durchflussmenge an Sauerstoff ist auf ein Bolusvolumen begrenzt. Nach Erreichen dieses Bolusvolumens steuert die Steuer- und Regeleinheit 18 das Magnetventil 10 schließend an.

Die Sauerstoffversorgungseinheit 6 weist zwei Druckgasflaschen 20 u. 20' als Sauerstoffspeicher auf, die für eine beliebige Anzahl von Druckgasflaschen stehen. An den Druckgasflaschen 20 u. 20' sind Rückschlagsicherungen 22 u. 22', gefolgt von elektrisch gesteuerten Absperrventilen 24 u. 24', angeordnet. Steuerleitungen 26 u. 26' verbinden die Absperrventile 24 u. 24' mit der elektronischen Steuer- und Regeleinheit 18, über die eines der Absperrventile 24 u. 24' öffnend und das bzw. die übrigen schließend angesteuert werden. Über Druckgasleitungen 28 u. 28' stehen die Druckgasflaschen 20 u. 20' mit Drucksensoren 30 u. 30' in Verbindung, die den Flaschendruck der Druckgasflaschen 20 u. 20' bzw. deren Befüllungsgrad aufnehmen. Zum Weiterleiten der von den Drucksensoren 30 u. 30' aufgenommenen Druckwerte sind die Drucksensoren 30 u. 30', über elektrische Leitungen 32 u. 32' mit der elektronischen Steuer- und Regeleinheit 18 verbunden.

Beim Aktivieren der Sauerstoffversorgungsvorrichtung schaltet die Steuer- und Regeleinheit 18 über die Steuerleitung 26 das Absperrventil 24 öffnend, sodass Sauerstoff der Druckgasflasche 20 über die Versorgungsleitung 4 der Impulsatemregelung 8 und der daran angeschlossenen Atemmaske 2 zugeführt wird. Während des Entleerens der Druckgasflasche 20 wird deren Flaschendruck von dem Drucksensor 30 aufgenommen. Nähert sich der Flaschendruck einer unteren Grenze, die durch den Entspannungsdruck des Sauerstoffs von z.B. 4 bar vorgegeben ist, d.h., die Druckgasflasche 20 verliert ihr Überdruckpotential gegenüber dem Umgebungsdruck, wird dies von der Steuer- und Regeleinheit 18 mittels des Drucksensors 30 erfasst. Die Steuer- und Regeleinheit 18 veranlasst das Öffnen des Absperrventils 24' und Sauerstoff der Druckgasflasche 20' wird in die Versorgungsleitung 4 in Richtung Impulsatemregelung 8 und Atemmaske 2 eingeleitet.

Zwischen den Absperrventilen 24 u. 24' der Druckgasflaschen 20 u. 20' und der Impulsatemregelung 8 ist in der Versorgungsleitung 4 eine elektronische Druckregelung vorgesehen, um den Flaschendruck der Druckgasflaschen 20 u. 20' von z.B. 200 bar auf einen Druck von ungefähr 4 bar eingangs der Impulsatemregelung 8 zu reduzieren.

Die elektronische Druckregelung weist druckgasflaschenseitig ein steuerbares Magnetventil 34 und zu Seiten der Impulsatemregelung 8 einen Zwischendruckspeicher 36 auf. Zwischen dem Magnetventil 34 und dem Zwischendruckspeicher 36 ist in der Versorgungsleitung 4 eine Drossel 38 angeordnet. An dem Zwischendruckspeicher 36 ist eine Leitung 40 angeschlossen, über die ein Drucksensor 42 angeschlossen ist. Das Magnetventil 34 ist über eine Steuerleitung 44 mit der elektronischen Steuer- und Regeleinheit 18 verbunden. Der Drucksensor 42 ist über die Leitung 45 an der Steuer- und Regeleinheit angeschlossen.

Ist die Sauerstoffversorgungsvorrichtung aktiviert, öffnet ein über die Steuerleitung 44 übermitteltes Steuersignal der elektronischen Steuer- und Regeleinheit 18 das Magnetventil 34. Sauerstoff aus einer der Druckgasflaschen 20 oder 20' kann gedrosselt über die Drossel 38 in den Zwischendruckspeicher 36 strömen. Der im Zwischendruckspeicher 36 herrschende Druck wird von dem Drucksensor 42 erfasst. Diese Werte werden über die Steuerleitung 44 an die Steuer- und Regeleinheit 18 weitergeleitet. Bei Erreichen eines Soll-Drucks von z.B. 4 bar veranlasst die Steuer- und Regeleinheit 18 das Sperren des Magnetventils 34. Durch die Atemtätigkeit des Benutzers an der Atemmaske 2 entleert sich der Zwischendruckspeicher 36, die Steuer- und Regeleinheit 18 veranlasst das erneute Öffnen des Magnetventils 34 zum Befüllen des Zwischendruckspeichers 36 und der soeben beschriebene Vorgang wiederholt sich. Dies ist allerdings nur beispielhaft zu verstehen, da keine zeitliche Abhängigkeit von der Sauerstoffentnahme aus dem Zwischendruckspeicher 36 durch Öffnen des Manetventils 10 und der Sauerstoffzufuhr zu dem Zwischendruckspeicher 36 durch Öffnen des Magnetventils 34 besteht. So ist es beispielsweise auch ein geöffnetes Magnetventil 34 während der Sauerstoffentnahme aus dem Zwischendruckspeicher 36 denkbar. Aufgrund des relativ großen Flaschendrucks der Druckgasflaschen 20 u. 20' sowie des relativ geringen Drucks und Volumens des Zwischendruckspeichers 36 sind Öffnungszeiten des Magnetventils 34 im Millisekundenbereich vorgesehen.

In einer alternativen Ausführungsform ist statt des Drucksensors 42 ein Druckschalter 52 vorgesehen, der über eine Leitung 40' mit dem Zwischendruckspeicher verbunden ist. Über die Steuerleitung 44 ist der Druckschalter 52 mit dem Magnetventil 34 und der Steuer- und Regeleinheit 18 leitungsverbunden. Bei Erreichen eines Soll-Drucks in dem Zwischendruckspeicher 36 wird das Magnetventil 34 von dem Druckschalter 52 die Versorgungsleitung 4 sperrend gestellt. Nach einer Druckabsenkung in dem Zwischendruckspeicher 36 schaltet der Druckschalter 52 das Magnetventil 34 wieder die Versorgungsleitung 4 öffnend. Der Speicherdruck in dem Zwischendruckspeicher 36 kann dadurch verändert werden, dass die Steuer- und Regeleinheit 18 über die Steuerleitung 44 an dem Druckschalter 52 den Schaltdruck entsprechend einstellt.

Neben der Steuerleitung 44 ist an dem Magnetventil 34 auch eine mechanische Zeitsteuerung 46 angeordnet. Diese ist für den Fall vorgesehen, dass das Magnetventil 34 von der Steuer- und Regeleinheit 18 fehlerhaft bzw. gar nicht angesteuert wird. Durch manuelles Betätigen der Zeitsteuerung 46 stellt diese in getakteten Zeitintervallen das Absperrventil 34 in eine öffnende und eine sperrende Stellung, sodass der Zwischendruckspeicher 36 über die Drossel 36 gleichmäßig mit Sauerstoff gefüllt wird, der dann über das in diesem Fall manuell betätigte und konstant geöffnete Magnetventil 10 der Impulsatemregelung 8 der Atemmaske 2 zur Verfügung gestellt wird.

Da bei Ausfall der elektronischen Steuerung des Magnetventils 34 eine Druckregelung des Sauerstoffdrucks in dem Zwischendruckspeicher 36 nicht mehr möglich ist, ist an der Versorgungsleitung 4 zwischen dem Zwischendruckspeicher 36 und der Impulsatemregelung 8 ein Überdruckventil 48 angeordnet, das bei etwaigem Überdruck den Leitungsdruck vor der Impulsatemregelung 8 auf zulässige Werte entspannt.

Insbesondere für den Einsatz der Sauerstoffversorgungsvorrichtung in der Luftfahrt, weist die Sauerstoffversorgungsvorrichtung ein Höhensensor 50 auf, der an der Steuer- und Regeleinheit 18 angebunden ist und der den Umgebungsdruck z.B. des Flugzeugs erfasst. Auf Grundlage der von dem Höhensensor 50 gelieferten Druckwerte veranlasst die Steuer- und Regeleinheit 18 in Relation zu einem Höhenanstieg eine Druckerhöhung in dem Zwischendruckspeicher 36 und oder eine längere Öffnungszeit des Magnetventils 10 der Impulsatemregelung 8. Auf diese Weise steht dem Benutzer der Atemmaske 2 eine der Flughöhe angepasste Sauerstoffmenge zur Verfügung. Alternativ zu dem eben beschriebenen barometrischen Höhensensor 50 ist es möglich, die Höhenbestimmung auch mittels anderer Messgrößen durchzuführen. Die Steuer- und Regeleinheit 18 ordnet dann den ermittelten Höhenwerten entsprechende Drücke des Zwischendruckspeichers 36 zu und steuert eine eventuelle Druckänderung.

### Bezugszeichenliste

- 2: Atemmaske
- 4: Versorgungsleitung
- 6: Sauerstoffversorgungseinheit
- 8: Impulsatemregelung
- 10: Magnetventil
- 12: Leitung
- 14: Drucksensor
- 16: Steuerleitung
- 18: Steuer- und Regeleinheit
- 20: Druckgasflasche
- 20': Druckgasflasche
- 22: Rückschlagsicherung
- 22': Rückschlagsicherung
- 24: Absperrventil
- 24': Absperrventil
- 26: Steuerleitung
- 26': Steuerleitung
- 28: Druckgasleitung
- 28': Druckgasleitung
- 30: Drucksensor
- 30': Drucksensor
- 32: Leitung
- 32': Leitung
- 34: Magnetventil
- 36: Zwischendruckspeicher
- 38: Drossel
- 40: Leitung
- 40': Leitung
- 42: Drucksensor
- 44: Steuerleitung
- 45: Leitung
- 46: Zeitsteuerung
- 48: Überdruckventil
- 50: Höhensensor
- 52: Druckschalter

## Patentansprüche

1. Sauerstoffversorgungsvorrichtung für eine Atemmaske (2) mit mindestens einem Sauerstoffdruckspeicher (20, 20'), der über ein steuerbares Absperrventil (34) mit einem Zwischenspeicher (36) leitungsverbunden ist, an den ein die Maske (2) versorgender Impulsatemregler (8) anschließt, wobei eine elektronische Steuerung (18) vorgesehen ist, welche das Absperrventil (34) in Abhängigkeit des im Zwischenspeicher (36) herrschenden Drucks steuert.

2. Sauerstoffversorgungsvorrichtung nach Anspruch 1, **dadurch gekennzeichnet, dass** die elektronische Steuerung (18) Teil einer elektronischen Steuer- und Regeleinrichtung (18) ist, welche auch ein Absperrventil (10) des Impulsatemreglers (8) steuert.

3. Sauerstoffversorgungsvorrichtung nach einem der vorangehenden Ansprüche, **dadurch gekennzeichnet, dass** die Steuer- und Regeleinrichtung (18) den Soll-Druck des Zwischenspeichers (36) höhen- oder druckabhängig einstellt.

4. Sauerstoffversorgungsvorrichtung nach einem der vorangehenden Ansprüche, **dadurch gekennzeichnet, dass** die Steuer- und Regeleinrichtung (18) die Schaltzeit des Absperrventils (10) des lmpulsatemreglers (8) höhen- oder druckabhängig einstellt.

5. Sauerstoffversorgungsvorrichtung nach einem der vorangehenden Ansprüche, **dadurch gekennzeichnet, dass** eine vorzugsweise manuell betätigbahre Notsteuereinrichtung (46) für das Absperrventil (34) vorgesehen ist, die das Absperrventil (34) taktweise öffnet und schließt.

6. Sauerstoffversorgungsvorrichtung nach einem der vorangehenden Ansprüche, **dadurch gekennzeichnet, dass** an oder in dem Zwischenspeicher (36) ein Drucksensor (42) angeordnet ist.

7. Sauerstoffversorgungsvorrichtung nach einem der vorangehenden Ansprüche, **dadurch gekennzeichnet, dass** an dem Sauerstoffdruckspeicher (20, 20') ein Drucksensor (30, 30') angeordnet ist.

8. Sauerstoffversorgungsvorrichtung nach einem der vorangehenden Ansprüche, **dadurch gekennzeichnet, dass** zwischen dem Zwischenspeicher (36) und dem Impulsatemregler (8) ein Überdruckventil (48) angeordnet ist.

9. Sauerstoffversorgungsvorrichtung nach einem der vorangehenden Ansprüche, **dadurch gekennzeichnet, dass** in der Leitungsverbindung (4) zwischen Absperrventil (34) und Zwischenspeicher (36) eine Drosselstelle (38) angeordnet ist.

10. Sauerstoffversorgungsvorrichtung nach einem der vorangehenden Ansprüche, **dadurch gekennzeichnet, dass** an der elektronischen Steuer- und Regeleinrichtung (18) ein Höhenmesser (50) angebunden ist.
